# EUROPÄISCHE PATENTANMELDUNG

(11) **EP 0 866 062 A1**
(43) Veröffentlichungstag der Anmeldung: **23.09.1998**
(21) Anmeldenummer: 98109084.8
(22) Anmeldetag: 01.02.1996
(51) Int. Cl.: C07D 285/16

(54) **Verfahren zur Herstellung von Ammoniumsalzen von 3-Isopropyl-2,1,3-benzothiadiazin-4-on-2,2-dioxid**

(30) Priorität: 15.02.1995 DE 19505036
(62) Teilanmeldung aus: 96902969.3
(71) Anmelder: BASF AKTIENGESELLSCHAFT, 67056 Ludwigshafen (DE)
(72) Erfinder: Merkle, Hans Rupert, Dr., 67061 Ludwigshafen (DE); Durein, Alfons, 67354 Römerberg (DE); Hansen, Hanspeter, Dr., 67061 Ludwigshafen (DE); Jäger, Karl-Friedrich, Dr., 67117 Limburgerhof (DE)

(57) **Zusammenfassung**

Verfahren zur Herstellung von Salzen von 3-Isopropyl-2,1,3-benzothiadiazin-4-on-2,2-dioxid der allgemeinen Formel I 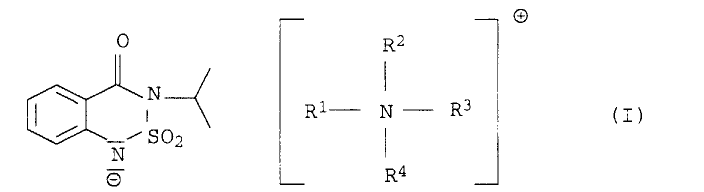 (R¹-R⁴ = H, niederes Alkyl, niederes Hydroxyalkyl) indem man Bentazon (IIa) in einem mit Wasser praktisch nicht mischbaren organischen Lösungsmittel mit einem Amin IIIa oder einem Ammoniumsalz IIIb umsetzt und das Salz I in Wasser aufnimmt oder indem man jeweils in Wasser Bentazon (IIa) mit einem Ammoniumsalz IIIb oder Natrium-Bentazon (IIb) mit einem Ammoniumsalz IIIc umsetzt.

## Beschreibung

Die vorliegende Erfindung betrifft Verfahren zur Herstellung von Salzen von 3-Isopropyl-2,1,3-benzothiadiazin-4-on-2,2-dioxid der allgemeinen Formel I 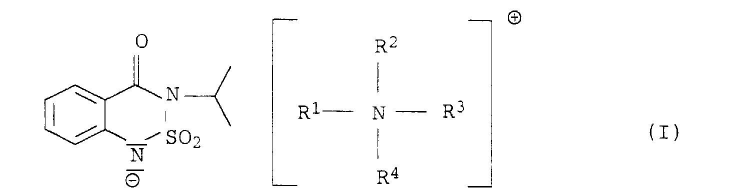 in der die Reste R¹, R², R³ und R⁴ unabhängig voneinander für Wasserstoff, niederes Alkyl oder niederes Hydroxyalkyl stehen.

Herbizide Benzothiadiazin-4-on-2,2-dioxide sind aus DE-A 15 42 836, DE-A 21 64 459 und DE-A 22 17 722 bekannt. Dort werden auch Ammoniumsalze als Anwendungsform genannt, wobei die Ammonium-, Methylammonium-, Trimethylammonium-, Ethylammonium-, Diethanolammonium- und Ethanolammoniumsalze besondere Erwähnung finden.

Es ist weiterhin allgemein bekannt, daß das Natrium-, das Calcium- und das Kaliumsalz von 3-Isopropyl-2,1,3-benzothiadiazin-4-on-2,2-dioxid (INN-Name: Bentazon) sehr hygroskopisch sind. Dies führt im Falle von Feststoff-Formulierungen dieser Salze dazu, daß das Produkt bereits durch Luftfeuchtigkeit verklumpt oder sogar zerfließt und dadurch nicht mehr ohne weiteres zu dosieren ist.

Beim Einbringen dieser Salze in wasserlösliche Folienbeutel werden letztere zudem durch Wechselwirkung der hygroskopischen Wirkstoffe mit den Folien dehydratisiert. Dies hat zur Folge, daß die Folien verspröden, d.h. ihre Lagerstabilität ist nicht mehr gewährleistet.

Bei der Synthese von Bentazon erhält man üblicherweise den Wirkstoff in neutraler Form und in der Regel gelöst in einem organischen Lösungsmittel (vgl. DE-A 27 10 382).

Meist überführt man den Wirkstoff anschließend in eines seiner Salze, da diese die Bioverfügbarkeit von Bentazon verbessern.

Aus der US-A 5 266 553 ist beispielsweise bekannt, Ammonium-Salze des Bentazons als fließfähige wasserlösliche Feststoffe zu formulieren. Dazu wird gemäß der allgemeinen Lehre des Patents zunächst eine wäßrige Mischung des Ammoniumsalzes hergestellt. Die Feststoff-Formulierung des Wirkstoffs erhält man hieraus durch Verdampfen des gesamten Lösungsmittels und Nachbehandlung des Produkts mit einer neutralisierenden Base. Der Verdampfungsschritt bei Mitverwendung von Wasser als Lösungsmittel erfordert jedoch einen hohen Energieaufwand und der Wirkstoff wird dabei sehr lange der erhöhten Verdampfungstemperatur ausgesetzt.

Der vorliegenden Erfindung lagen daher Verfahren zur Herstellung von Ammoniumsalzen des Bentazons zugrunde, bei denen die Nachteile des vorstehend beschriebenen Herstellungsverfahrens ganz oder teilweise vermieden werden.

Demgemäß wurde ein Verfahren zur Herstellung von Salzen von 3-Isopropyl-2,1,3-benzothiadiazin-4-on-2,2-dioxid der allgemeinen Formel I 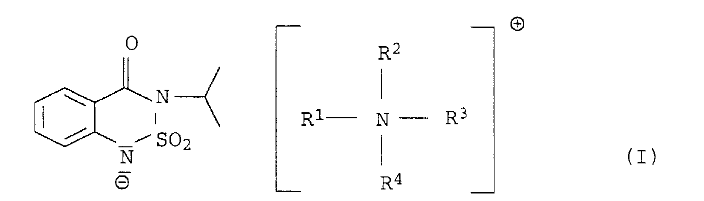 in der die Reste R¹, R², R³ und R⁴ unabhängig voneinander für Wasserstoff, niederes Alkyl oder niederes Hydroxyalkyl stehen, gefunden, welches dadurch gekennzeichnet ist, daß man
a) 3-Isopropyl-2,1,3-benzothiadiazin-4-on-2,2-dioxid (IIa) 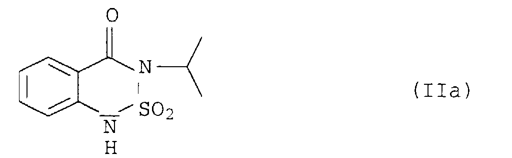 in einem mit Wasser praktisch nicht mischbaren organischen Lösungsmittel und gewünschtenfalls in Gegenwart von Wasser mit einem Amin der allgemeinen Formel IIIa 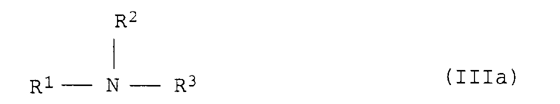 oder einem Ammoniumsalz der allgemeinen Formel IIIb 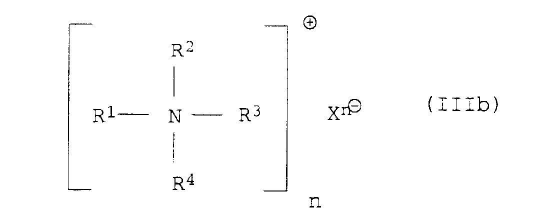 worin X das Anion einer Säure vom pKₛ-Wert größer 4 oder das Hydroxylion bedeutet und n gleich ist der Zahl der negativen Ladungen des Anions X, umsetzt und
b) das Salz I in Wasser aufnimmt. Darüber hinaus wurde ein Verfahren zur Herstellung von Salzen von 3-Isopropyl-2,1,3-benzothiadiazin-4-on-2,2-dioxid der allgemeinen Formel I 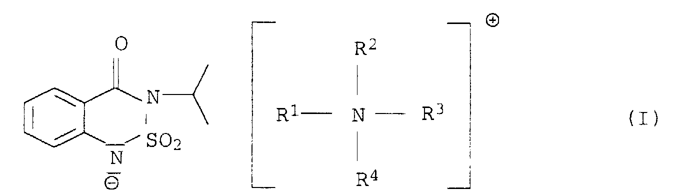 in der die Reste R¹, R², R³ und R⁴ unabhängig voneinander für Wasserstoff, niederes Alkyl oder niederes Hydroxyalkyl stehen, gefunden, welches dadurch gekennzeichnet ist, daß man
a) 3-Isopropyl-2,1,3-benzothiadiazin-4-on-2,2-dioxid (IIa) 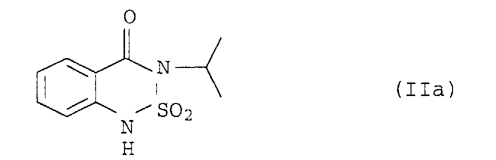 in Wasser mit einem Ammoniumsalz der allgemeinen Formel IIIb 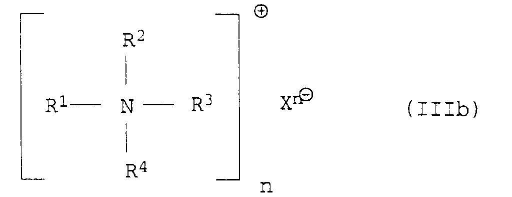 worin X das Anion einer Säure mit einem pKₛ-Wert größer 4 oder das Hydroxylion bedeutet und n gleich ist der Zahl der negativen Ladungen des Anions X, oder
b) das Natriumsalz von Bentazon (IIb) 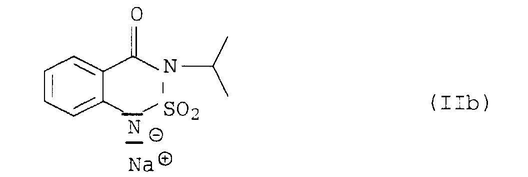 in Wasser mit einem Ammoniumsalz der allgemeinen Formel IIIc 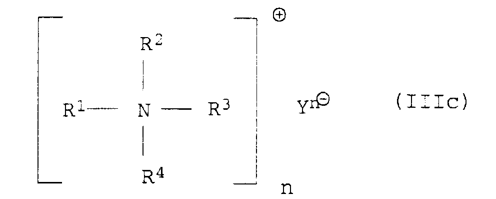 worin Y das Anion einer Säure bedeutet und n gleich ist der Zahl der negativen Ladungen des Anions Y, umsetzt.

Unter niederem Alkyl bzw. niederem Hydroxyalkyl werden Alkylgruppen bzw. Hydroxyalkylgruppen mit bis zu 8, vorzugsweise mit bis zu 6 C-Atomen wie Methyl, Hydroxymethyl, Ethyl, 2-Hydroxyethyl, Propyl, 3-Hydroxypropyl und Butyl verstanden.

Die erfindungsgemäßen Verfahren werden im folgenden als Verfahren A, und B bezeichnet.

### Verfahren A

In diesem Verfahren wird Bentazon (IIa) in einem mit Wasser praktisch nicht mischbaren organischen Lösungsmittel mit einem Amin IIIa oder einem Ammoniumsalz IIIb, gewünschtenfalls in Gegenwart von Wasser, umgesetzt und das Salz I in Wasser aufgenommen (s. Schema 1).

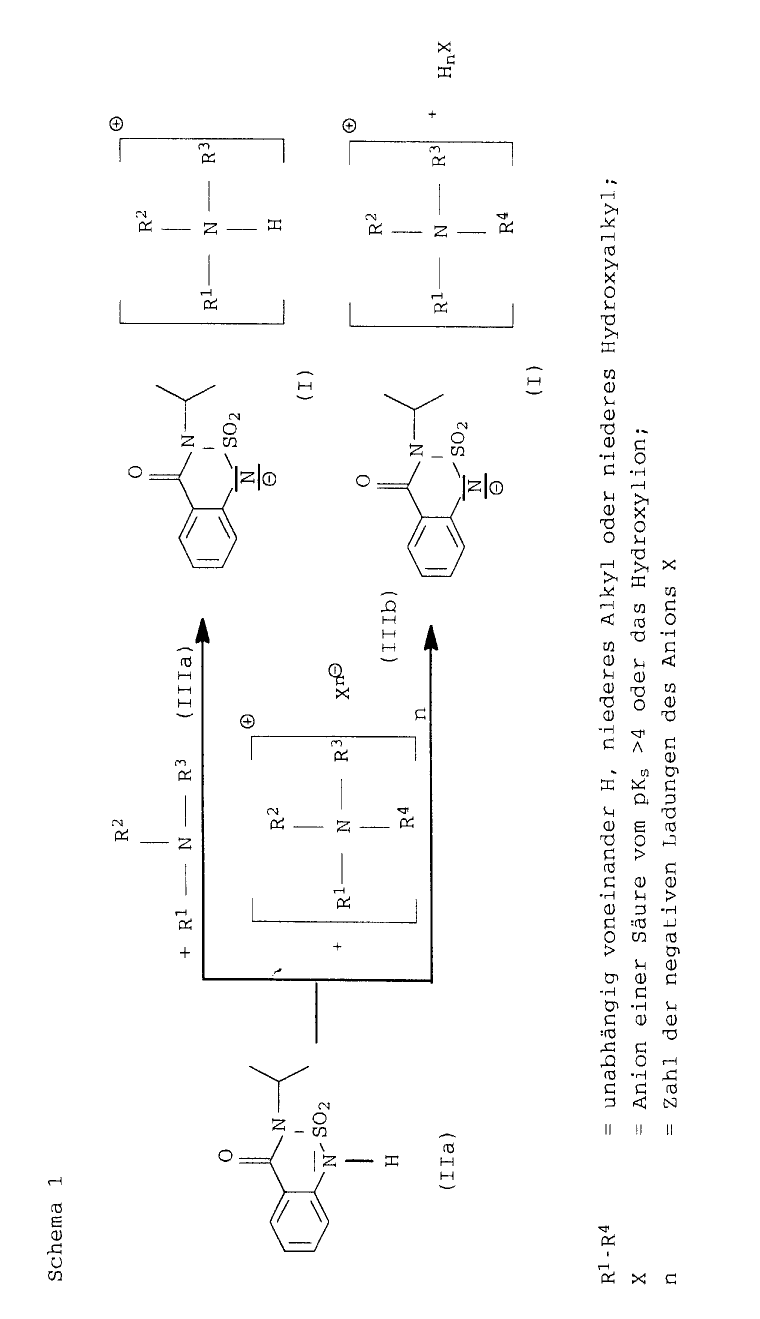

Die Amine IIIa sind allgemein bekannt. Das gleiche gilt für die Ammoniumsalze IIIb (vgl. Houben-Weyl, Methoden der Organischen Chemie, 4. Auflage, Thieme Verlag, Stuttgart, Band 11/2, Seite 591 ff.).

Als Anion X in der allgemeinen Formel IIIb sind das Carbonation und das Hydrogencarbonation bevorzugt und das Hydroxylion besonders bevorzugt.

In der Regel wird das Amin IIIa oder das Ammoniumsalz IIIb in einer äquimolaren Menge, bezogen auf Bentazon (IIa), eingesetzt. Es kann zur Vervollständigung der Umsetzung vorteilhaft sein, das Amin IIIa bzw. das Ammoniumsalz IIIb in einem Überschuß zu verwenden. Dieser Überschuß braucht aber zur Erzielung eines vollständigen Umsatzes im allgemeinen 10 mol-%, bezogen auf IIa, nicht zu übersteigen.

Als mit Wasser praktisch nicht mischbare organische Lösungsmittel kommen in Betracht: Alkane, vorzugsweise mit 5 bis 8 C-Atomen, vor allem n-Alkane wie n-Pentan und n-Hexan und Halogenkohlenwasserstoffe, vorzugsweise Halogenalkane wie Mono- und Dichloralkane mit 2 bis 4 C-Atomen wie 1,1-Dichlorethan, 1,3-Dichlorpropan, 1,2-Dichlorpropan und insbesondere 1,2-Dichlorethan.

Ferner eignen sich auch Gemische von zwei oder mehreren dieser mit Wasser praktisch nicht mischbaren organischen Lösungsmittel.

Besonders bevorzugt als mit Wasser praktisch nicht mischbares organisches Lösungsmittel ist 1,2-Dichlorethan für sich allein.

Bezogen auf ein Mol Bentazon (IIa) verwendet man normalerweise 1 bis 4 und vor allem 1,5 bis 3 kg Lösungsmittel.

Das Verfahren kann bei Temperaturen von 20 bis 80°C durchgeführt werden. Die Temperatur bei der Umsetzung hat einen Einfluß vor allem auf die Löslichkeit des Bentazons (IIa), welche mit der Temperatur zunimmt.

Vor allem in denjenigen Fällen, wo ein gasförmiges oder niedrigsiedendes Amin IIIa beteiligt ist, sollte die Temperatur jedoch einen Wert von 60°C nicht überschreiten. Vorzugsweise führt man die Umsetzung von Bentazon IIa mit den Aminen IIIa bzw. den Ammoniumsalzen IIIb bei Temperaturen von 20 bis 60, und insbesondere von 25 bis 50°C durch.

Die Umsetzung wird im allgemeinen bei einem Druck von 0,5 bis 10, vorzugsweise 1 bis 3 bar und insbesondere bei Normaldruck (Atmosphärendruck) durchgeführt.

Als Reaktoren eignen sich die üblicherweise für derartige Umsetzungen geeigneten Vorrichtungen.

Das gebildete Salz I wird in Wasser aufgenommen, wobei dieses bereits während der Reaktion oder erst bei Reaktionsende zugesetzt werden kann. Werden dabei geringe Mengen des organischen Lösungsmittels mit der Wasserphase abgetrennt, so können diese vor der Isolierung des Salzes I in an sich bekannter Weise, etwa durch Strippen oder gegebenenfalls durch azeotrope Destillation - etwa im Falle von 1,2-Dichlorethan/Wasser als Reaktionsmedium - bei Normaldruck oder vermindertem Druck entfernt werden.

Um das Salz vollständig aufzunehmen, verwendet man in der Regel, bezogen auf kg des Salzes I, 1 bis 5, vorzugsweise 2 bis 4 und insbesondere 2,5 bis 3,5 kg Wasser. Meist fällt das Salz I bereits bei der Reaktionstemperatur aus. Zur Vervollständigung der Fällung wird die Lösung meist abgekühlt. Die Kristallisation erfolgt vorzugsweise bei 5 bis 40 und insbesondere bei 15 bis 25°C.

Ein besonderer Vorteil des erfindungsgemäßen Verfahrens A liegt darin, daß auf diese Weise das organische Lösungsmittel, nachdem es von der Wasserphase abgetrennt worden ist, unmittelbar für weitere Umsetzungen verwendet werden kann, ohne daß es teilweise oder vollständig zum Zwecke der Produktisolierung verdampft und/ oder durch Destillation gereinigt werden muß.

Mit dem Verfahren A läßt sich, bei Rückführung der Mutterlauge, das Salz I in der Regel mit einer Ausbeute von 98 bis 100 % bei einer Reinheit von mindestens 98 % gewinnen.

Das Verfahren A eignet sich im besonderen Maße zur Herstellung des NH₄⁺-Salzes von Bentazon (I; R¹⁻R⁴ = H).

### Verfahren B

Im Verfahren B wird Bentazon (IIa) in Wasser mit einem Ammoniumsalz IIIb oder das Natriumsalz von Bentazon (IIb) in Wasser mit einem Ammoniumsalz IIIc umgesetzt (s. Schema 2).

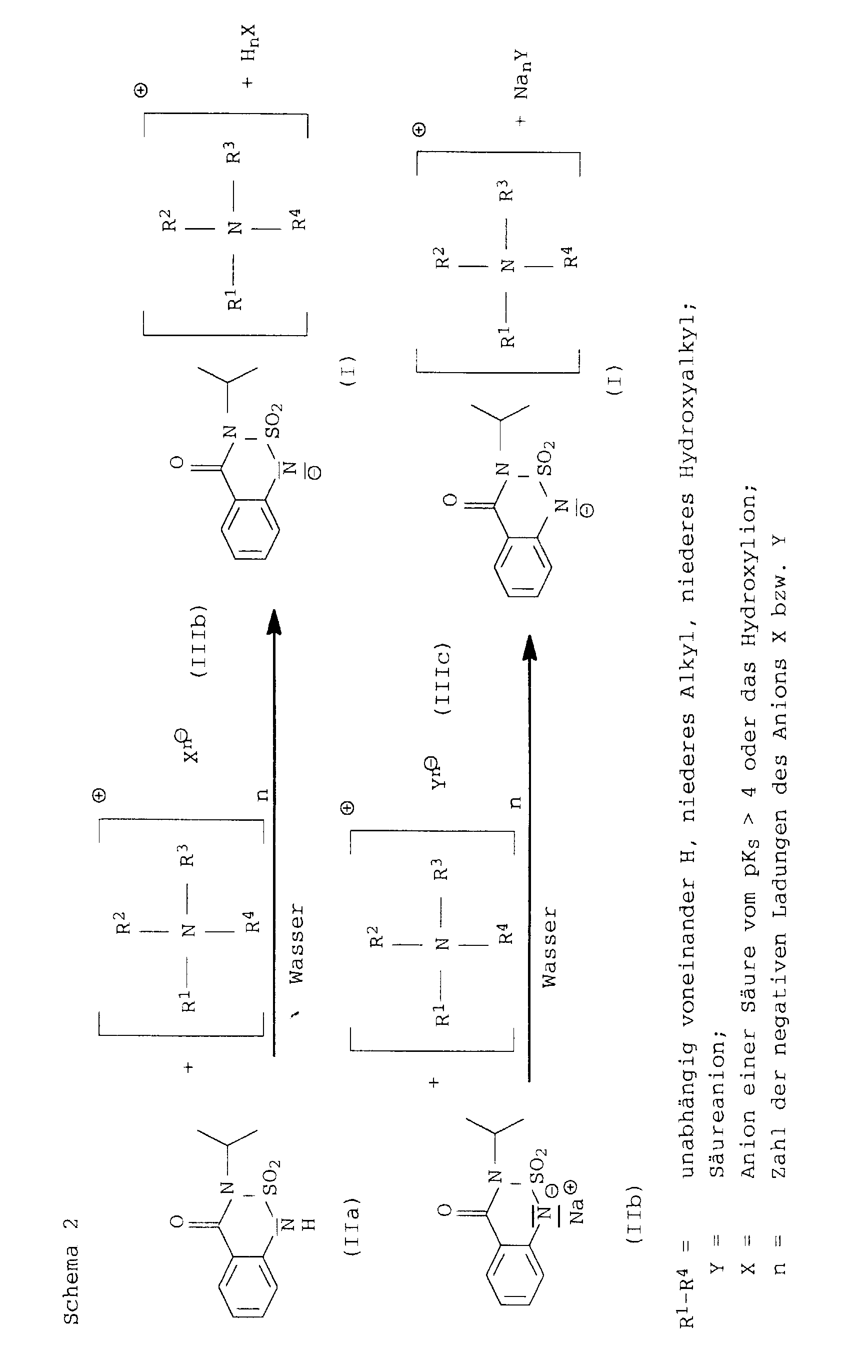

Das Verfahren B eignet sich im besonderen Maße zur Herstellung des NH₄⁺-Salzes von Bentazon (I; R¹-R⁴=Wasserstoff).

Die Ammoniumsalze IIIb sind allgemein bekannt (vgl. Houben-Weyl, Methoden der Organischen Chemie, 4. Auflage, Thieme Verlag, Stuttgart, Band 11/2, Seite 591 ff.).

Als Anionen Y in der allgemeinen Formel IIIc kommen in Betracht: Sulfat, Hydrogensulfat, Phosphat, Hydrogenphosphat oder Dihydrogenphosphat, vorzugsweise Halogenid oder Acetat und insbesondere Chlorid, Nitrat, Formiat, Carbonat und Hydrogencarbonat.

Als Säureanion Y in der allgemeinen Formel IIIc bzw. als Anion X einer Säure vom pKs-Wert > 4 sind das Carbonation und das Hydrogencarbonation bevorzugt und für X ist das Hydroxylion besonders bevorzugt.

In der Regel wird das Ammoniumsalz IIIb, bezogen auf Bentazon (IIa), und das Ammoniumsalz IIIc, bezogen auf das Natriumsalz von Bentazon (IIb), in einer äquimolaren Menge eingesetzt. Es kann zur Vervollständigung der Umsetzung vorteilhaft sein, die Ammoniumsalze in einem Überschuß zu verwenden. Dieser Überschuß braucht aber zur Erzielung eines vollständigen Umsatzes im allgemeinen 10 mol-%, bezogen auf IIa bzw. IIb, nicht zu übersteigen.

Bezogen auf ein Mol Bentazon (IIa) oder dessen Natriumsalz IIb verwendet man normalerweise 0,2 bis 4 und vor allem 0,2 bis 2 kg Wasser.

Das Natriumsalz NaₙY ist in Wasser gewöhnlich besser löslich als das Salz I. Verbleibt letzteres daher teilweise gelöst, so kann seine Abtrennung durch (fraktionierte) Kristallisation erfolgen. Diese Arbeitsweise ist dem Fachmann geläufig, so daß sich weitere Ausführungen hierzu erübrigen.

Zur Erzielung einer guten Ausbeute bei der Kristallisation hat sich ein Molverhältnis von Wasser zu Salz I von 50 zu 1 bis 30 zu 1 besonders bewährt.

Das Verfahren kann bei Temperaturen von 10 bis 80°C durchgeführt werden. Die Temperatur bei der Umsetzung hat einen Einfluß vor allem auf die Löslichkeit des Bentazons (IIa) und dessen Natriumsalzes IIb, welche mit der Temperatur zunimmt. Vorzugsweise führt man die Umsetzung bei Temperaturen von 20 bis 70, und insbesondere von 40 bis 60°C durch.

Die Umsetzung wird im allgemeinen bei einem Druck von 0,5 bis 10, vorzugsweise 1 bis 3 bar und insbesondere bei Normaldruck (Atmosphärendruck) durchgeführt.

Als Reaktoren eignen sich die üblicherweise für derartige Umsetzungen geeigneten Vorrichtungen.

Mit dem Verfahren B lassen sich die Salze I in der Regel mit einer Ausbeute von größer 80 % bei einer Reinheit von mindestens 98 % gewinnen. Durch Rückführung der Mutterlauge kann eine Ausbeute von mehr als 98 % erreicht werden.

Das nach einem der Verfahren A bis B hergestellte Salz I kann in an sich bekannter Weise isoliert werden. Dort wo es bereits aus der Reaktionsmischung auskristallisiert, geschieht dies vor allem durch Filtration. Sofern das Salz in gelöster Form anfällt, kann die gesamte Lösung nach allgemein bekanntem Verfahren, etwa durch Eindampfen, vor allem bei vermindertem Druck, vom Lösungsmittel befreit werden.

Das nach dem Verfahren B erhältliche Salz I, welches aus einer wäßrigen Phase kristallisiert wurde, enthält in der Regel bereits weniger als 10 Gew.-% Wasser.

Das (von organischem Lösungsmittel oder Wasser) feuchte Salz I, wird in der Regel bei einer Temperatur von 20 bis 80, vorzugsweise 40 bis 60°C getrocknet. Die Trocknung kann in üblichen Trokkenvorrichtungen erfolgen. Bevorzugt arbeitet man bei vermindertem Druck oder unter Erwärmen des Produktes I im Luftstrom.

Die Mutterlaugen, welche nach Abtrennung von kristallisiertem Salz I zurückbleiben, enthalten in manchen Fällen noch bis zu 20 % des Salzes I in gelöster Form. Wo es gewünscht wird, kann die Isolierung dieses gelösten Wirkstoffs in an sich bekannter Weise, etwa durch Einengen der Lösung und erneute Kristallisation oder durch vollständiges Eindampfen der Mutterlauge erfolgen. Häufig kann die Mutterlauge auch wieder in das Verfahren zurückgeführt werden.

Granulate aus Lösungen der Salze I erhält man ausgehend von den bei der Herstellung anfallenden Lösungen oder erhält man den Kristallisations-Mutterlaugen vorzugsweise nach einem Wirbelbett-Verfahren oder durch die Agglomeration an ein Pulver von I, welches seinerseits durch Sprühtrocknung oder Vakuumtrocknung hergestellt wurde.

Die so erhaltenen Granulate bestehen üblicherweise zu 20 bis 100 Gew.-% aus Salz I. Die Korngröße dieser Granulate liegt im allgemeinen bei 200 µm bis 3000 µm. Der Staubanteil der Granulate ist gering. Der Staubgehalt einer 30 g-Probe ist kleiner als 20 mg (CIPAC MT 171: "Dustiness of Granular Formulation") wodurch eine hohe Sicherheit für den Anwender erreicht wird. Das Schüttgewicht derartiger Granulate liegt in der Regel bei 400-800 g/l.

Die Salze I zeigen ein ausgezeichnetes Lagerverhalten in wasserlöslichen Folienbeuteln. Derartige Folienbeutel sind an sich bekannt (EP-A 449 773, EP-A 493 553), so daß sich weitere Ausführungen hierzu erübrigen.

Die gefüllten Folienbeutel enthalten üblicherweise 0,1 bis 10 kg, vorzugsweise 0,5 bis 5 kg an Wirkstoff I. Die Stärke der Folien beträgt 20 bis 100 µm, vorzugsweise 30 bis 60 µm. Der Wassergehalt in den polymeren Folien kann bis zu 20 Gew.-% betragen.

Die wie vorstehend beschrieben erhaltenen Granulate oder die gefüllten Folienbeutel können außer den Salzen I noch weitere übliche Zusatzstoffe, z.B. oberflächenaktive Stoffe, Füllstoffe oder auch weitere Pflanzenschutzwirkstoffe enthalten.

Es wurde gefunden, daß die Salze I, und vor allem das NH₄⁺-Salz von Bentazon, in den jeweiligen Reaktionsmedien eine relativ geringe Löslichkeit aufweisen, verglichen mit den Ausgangsstoffen IIa bzw. IIb. Dieser Effekt wird in den erfindungsgemäßen Verfahren genutzt, um das Produkt I in einfacher Weise in fester Form aus dem Reaktionsgemisch zu isolieren.

Die Ammoniumsalze und besonders das NH₄⁺-Salz lösen sich hingegen, verglichen mit dem zumeist verwendeten Natriumsalz, deutlich schneller in Wasser auf, und damit verringert sich der Aufwand für die Zubereitung wäßriger Wirkstoffbrühen.

### Beispiele

### Beispiel 1

In eine Lösung von 24 g Bentazon (IIa) in 2376 g 1,2-Dichlorethan wurde unter Rühren bei einer Temperatur von 20-50°C 1,7-3 g Ammoniak eingegast, wobei sich eine Suspension bildete. Der Feststoff wurde bei 20°C durch Filtration abgetrennt und bei vermindertem Druck von Lösungsmittelresten befreit. Man erhielt 25,4 g Ammoniumbentazon (Fp. 180°C).

### Beispiel 2

In eine Suspension von 24 g Bentazon (IIa) und 300 g Wasser wurden unter Rühren 4,8 g Ammoniumcarbonat eingetragen. Das Reaktionsgemisch wurde 2 Stunden bei 50°C nachgerührt und durch Filtration von Feststoffteilchen befreit. Nach dem Eindampfen der Lösung bei vermindertem Druck verblieben 25,5 g Ammonium-Bentazon.

### Beispiel 3

Es wurde wie in Beispiel 2 gearbeitet, jedoch wurden anstelle des Ammoniumcarbonats 7,9 g Ammoniumhydrogencarbonat verwendet. Es wurden 25,5 g Ammonium-Bentazon erhalten.

### Beispiel 4

In eine Lösung von 26,3 g Natrium-Bentazon in 21,7 g Wasser wurden bei 50°C unter Rühren 8 g Ammoniumnitrat eingetragen, und das Reaktionsgemisch wurde noch eine Stunde nachgerührt. Nach dem Abkühlen auf 20°C wurde der Niederschlag abfiltriert, zweimal mit jeweils 5 ml Eiswasser gewaschen und bei vermindertem Druck und 50°C getrocknet. Es wurden 18,9 g Ammoniumbentazon von 99% Reinheit erhalten.

### Beispiel 5

Es wurde wie in Beispiel 4 beschrieben gearbeitet, jedoch wurden anstatt des Ammoniumnitrats 6,3 g Ammoniumformiat eingesetzt. Die Ausbeute an Ammoniumbentazon betrug 21 g. Das Produkt hatte eine Reinheit von 98,4%.

### Beispiel 6

Eine Lösung von 24 g Bentazon (IIa) in 216 g 1,2-Dichlorethan wurde unter Rühren bei einer Temperatur von 50-60°C mit 34 g Ammoniakwasser (5 %-ige Lösung von Ammoniak in Wasser) versetzt. Nach beendeter Zugabe wurde die wäßrige Phase bei 50-60°C abgetrennt. Beim Erkalten der wäßrigen Phase fiel das Ammoniumbentazon kristallin aus. Der Feststoff wurde bei 20°C durch Filtration abgetrennt und bei vermindertem Druck und einer Temperatur von 50°C von Lösungsmittelresten befreit. Man erhielt 11,8 g an Ammoniumbentazon (Fp. 180°C). Aus der Mutterlauge erhielt man durch Verdampfen des Wassers bei vermindertem Druck und 50-60°C weitere 13,7 g an Ammoniumbentazon.

### Beispiel 7

Eine Lösung von 24 g Bentazon (IIa) in 216 g 1,2-Dichlorethan wurde unter Rühren bei einer Temperatur von 30-50°C mit 22,5 g einer 20 %-igen wäßrigen Lösung von Dimethylamin versetzt. Nach beendeter Zugabe wurde die wäßrige Phase bei 50-60°C abgetrennt und bei vermindertem Druck und 50-60°C zur Trockne eingedampft. Man erhielt 28 g Dimethylammoniumbentazon (Fp. 145-147°C; Reinheit > 99% nach HPLC-Analyse für Bentazon und Titration für Dimethylammonium).

### Beispiel 8

Eine Mischung aus 24 g Bentazon (IIa), 4,8 g Ammoniumcarbonat, 220 g 1,2-Dichlorethan und 300 g Wasser wurde 1 Stunde bei 50-60°C gerührt. Danach wurden die Phasen getrennt, und das Wasser wurde bei vermindertem Druck und 50-60°C entfernt. Es wurden 25,5 g Ammoniumbentazon erhalten.

### Beispiel 9

Entsprechend Beispiel 8, jedoch unter Verwendung von 7,9 g Ammoniumhydrogencarbonat erhielt man 25,5 g Ammoniumbentazon.

### Beispiel 10

Eine 20 %-ige wäßrige Lösung von Ammoniumbentazon wurde auf einem Wirbelschichtsprühgranulator bei 120°C Trocknungsluft-Temperatur getrocknet. Dabei wurde die Ammoniumsalzlosung eingedüst und es entstanden Granulatpartikel durch Agglomeration und Trocknung. Das erhaltene Granulat enthielt 99,6 Gew.-% an Ammoniumbentazon und hatte einen Restgehalt an Wasser von 0,4 Gew.-%. Die mittlere Korngröße im Granulat betrug 0,3 mm (maximaler Durchmesser). Das erhaltene Granulat war staubfrei und löste sich schnell in Wasser. Es war außerdem nicht hygroskopisch, d.h. es blieb auch bei längerer Aufbewahrung an feuchter Luft fließfähig.

### Beispiel 11

Ein Wirbelschichtsprühgranulator wurde mit 75 g Ammoniumsulfat-Pulver beschickt. Danach wurde in den so vorbereiteten Granulator 375 g einer 20 gew.-%ige wäßrige Lösung von Ammoniumbentazon bei 120°C Trocknungsluft-Temperatur eingedüst. Es entstanden Granulatpartikel durch Agglomeration und Trocknung. Das erhaltene Granulat enthielt 50 Gew.-% an Ammoniumbentazon und hatte einen Restgehalt an Wasser von 0,1-0,5 Gew.-%. Die mittlere Korngröße im Granulat betrug 1-2 mm (maximaler Durchmesser) . Das erhaltene Granulat war staubfrei und löste sich schnell in Wasser. Es war außerdem nicht hygroskopisch, d.h. es blieb auch bei längerer Aufbewahrung an feuchter Luft fließfähig.

### Beispiel 12

### Physikalisches Verhalten der Produkte

### a) Untersuchung zur Hygroskopizität der Salze

Jeweils 1 g der Probe wurde für 48 Stunden bei 50°C im Vakuum getrocknet. Die getrockneten Proben wurden bei 55% und 65% relativer Luftfeuchte und 20°C Temperatur aufbewahrt und die Gewichtszunahme der Proben nach Erreichen des Gleichgewichtszustandes gemessen. Ebenso wurden die Fließeigenschaften der Proben und deren Aussehen beurteilt. Hinsichtlich der Hygroskopizität kritische Substanzen nahmen viel Wasser aus der Luft bis zum Erreichen des Gleichgewichtszustandes auf. Dies führte zum Verbacken der Substanzen. Die Ergebnisse sind in der folgenden Tabelle zusammengestellt.

| Art des Salzes | rel. Luftfeuchte [%] | Gewichtszunahme [%] | Eigenschaften nach der Lagerung |
|---|---|---|---|
| Natriumsalz | 55 | 12,6 % | verklumpt, verbacken |
| Kaliumsalz | 55 | 6,7 % | verklumpt, verbacken |
| Kaliumsalz | 55 | 12,0 % | verklumpt, verbacken |
| Ammoniumsalz | 55 | 0,5 % | kristallin, fließfähig |
| | 65 | 0,5 % | kristallin, fließfähig |

### b) Untersuchung zum Verhalten der Salze im Folienbeutel:

Jeweils 10 g an Substanz in Form eines Granulats wurden in Folienbeutel eingeschweißt. Die gefüllten Folienbeutel (Folie: Monosol 8030, Hersteller: Chris Craft Inc., USA) wurden dann 4 Wochen bei verschiedenen Temperaturen in wasserdampfdichter Umverpackung aufbewahrt. Die Stabilität der Folien drückte sich durch die Elastizität der Folien bei mechanischer Beanspruchung aus. Wenn durch das Bentazon-Salz Wasser aus der Folie aufgenommen wurde, wurde die Folie spröde. Beispielsweise verlor die Folie Monosol 8030 in Gegenwart von Natrium-Bentazon in einem geschlossenen Behältnis einen großen Teil der im Film enthaltenen Restfeuchte. Bei Raumtemperatur ging diese von anfänglich 14% auf 6% im Gleichgewichtszustand zurück. Die Folge war eine Versprödung des Films und ein Platzen des Beutels bei mechanischer Belastung wie Transport, Stoßen und Belasten. Die Ergebnisse von Modellversuchen sind in der folgenden Tabelle zusammengefaßt.

| | | |
|---|---|---|
| Art des Salzes | T | Eigenschaften des Folienbeutels |
| Natriumsalz | 20 | spröde, brüchig |
| | 30 | spröde, brüchig |
| Ammoniumsalz | 20 | elastisch, stabil |
| | 30 | elastisch, stabil |

## Patentansprüche

1. Verfahren zur Herstellung von Salzen von 3-Isopropyl-2,1,3-benzothiadiazin-4-on-2,2-dioxid der allgemeinen Formel I 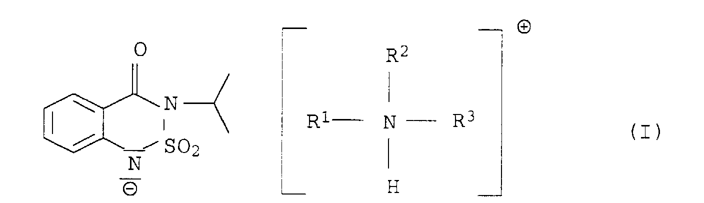 in der die Reste R¹, R^{2,} R³ und R⁴ unabhängig voneinander für Wasserstoff, niederes Alkyl oder niederes Hydroxyalkyl stehen, dadurch gekennzeichnet, daß man
a) 3-Isopropyl-2,1,3-benzothiadiazin-4-on-2,2-dioxid (IIa) 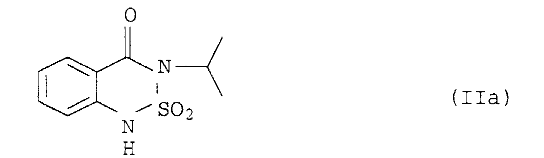 in einem mit Wasser praktisch nicht mischbaren organischen Lösungsmittel und gewünschtenfalls in Gegenwart von Wasser mit einem Amin der allgemeinen Formel IIIa 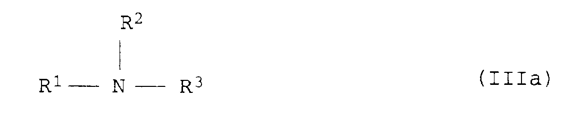 oder einem Ammoniumsalz der allgemeinen Formel IIIb 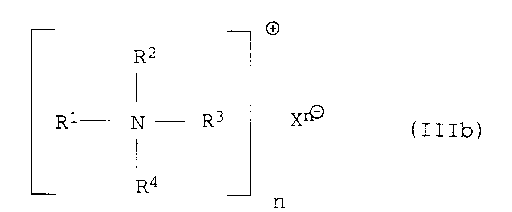 worin X das Anion einer Säure vom pKₛ-Wert größer 4 oder das Hydroxylion bedeutet und n gleich ist der Zahl der negativen Ladungen des Anions X, umsetzt und
b) das Salz I in Wasser aufnimmt.

2. Verfahren nach Anspruch 1, dadurch gekennzeichnet, daß man als organisches Lösungsmittel 1,2-Dichlorethan verwendet.

3. Verfahren nach Anspruch 1 oder 2, dadurch gekennzeichnet, daß man ein Amin IIIa oder Ammoniumsalz IIIb einsetzt, in denen die Reste R¹ bis R⁴ für Wasserstoff stehen.
